(19) Europäisches Patentamt
European Patent Office
Office européen des brevets

(11) **EP 4 144 826 A1**

(12) **EUROPEAN PATENT APPLICATION**

(43) Date of publication:
**08.03.2023 Bulletin 2023/10**

(21) Application number: **21306217.7**

(22) Date of filing: **06.09.2021**

(51) International Patent Classification (IPC):
*C12M 1/00* (2006.01)          *C12M 1/26* (2006.01)
*C12M 1/42* (2006.01)

(52) Cooperative Patent Classification (CPC):
**C12M 35/04; C12M 29/04; C12M 33/00**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Kolibri**
**75116 Paris (FR)**

(72) Inventors:
• DUMY, Gabriel
  **75011 PARIS (FR)**
• QUILICHINI, Amélie
  **75016 PARIS (FR)**

(74) Representative: **Atout PI Laplace**
**Immeuble "Visium"**
**22, avenue Aristide Briand**
**94117 Arcueil Cedex (FR)**

(54) **CELL CULTURE SYSTEM COMPRISING COMPARTMENTS AND AN ACOUSTIC ACTUATION DEVICE AND METHODS THEREOF**

(57) The invention relates to a cell culture system comprising a culture chamber comprising a flow compartment and a culture compartment being separated by a membrane, wherein the membrane is permeable to gas and is acoustically transparent, at least one inlet and one outlet to the flow compartment, at least one inlet and one outlet to the culture compartment; an acoustic actuation device comprising at least a first element being an ultrasonic transducer and an opposed second element being a reflector or a second ultrasonic transducer, wherein the acoustic actuation device is arranged to generate an acoustic field in the culture compartment. The invention also relates to a method for culturing cells using the cell culture system of the invention.

**FIG 1**

EP 4 144 826 A1

**Description**

FIELD OF THE INVENTION

The present invention relates to a cell culture system and methods thereof. Notably, the present invention relates to a cell culture system comprising a culture chamber which comprises a flow compartment and a culture compartment, the cell culture system further comprising an acoustic actuation device. The present invention relates to a method thereof for culturing cells using such cell culture system by maintaining with the acoustic actuation device at least one cell introduced in the culture compartment.

BACKGROUND PRIOR ART

More and more complex biologics are being exploited in modern pharmacopoeia such as antibodies, stem cells, viral vectors, etc. These biologics are not manufactured by chemical synthesis, i.e. through the reaction of several chemical molecules, but by living cells. Therefore, these cells need to be cultured in mass to reach the required quantities. These cells are usually mammalian cell lines, for which the historic means of culture are bioreactors. Alternatively, insect cells have also been used in conjunction with the baculovirus expression vector system. A bioreactor is a device that allow for the culture of a biological objects automatically, in a growth volume. Usually, these devices comprise a culture medium tank where agitation, culture, oxygenation, and pH regulation take place. The biological objects are usually included in the culture medium, where their whole growth cycle takes place.

However these bioreactors are designed for monocellular organisms such as yeasts and bacteria and are poorly adapted for culturing cells of multicellular organisms. The issue is that the cells of multicellular organisms are fragile and are not used to being grown on their own. Traditional mammalian cells usually grow surrounded by their peers, and usually in a well-regulated organism such as humans.

In addition to that, most of the bioproduction capacities that are adapted to these monocellular organisms have evolved from bacterial and yeast bioreactors, which are cells well suited to suspension culture in hostile environments. On the contrary, cells from multicellular organisms and in particular mammalian cells are adherent cells, meaning that they usually grow attached to a substrate. There is thus a technological gap between a demand and the reality of biology in this domain.

Adherent culture techniques have been developed, usually by repeating in parallel a simple validated mean of culture. For instance some techniques use an adherent support and multilayer/vertical stacking of culture surfaces. However these techniques do not allow a sufficient production to meet the biologics demand.

Antibodies being historically the first biologic produced on a massive scale, most of the mammalian cell bioreactor literature reports to them. However, traditional bioreactors are not suitable with all the cells lines that are able to produce biologics of interest and some adaptation have to be made since these ones are not adapted for instance to suspension culture.

A first solution is to design cell lines that are adapted in a culture in suspension in order to increase efficiency. Starting with an adherent cell line, it is generally possible by adjusting the culture and feeding conditions to generate a mutant clone that will be adapted to suspension growth (Berg DT et al, High-level expression of secreted proteins from cells adapted to serum-free suspension culture. Biotechniques. 1993 Jun ; Mcallister, R. et al, Adaptation of Recombinant HEK-293 Cells to Growth in Serum Free Suspension, Animal Cell Technology: Products from Cells, Cells as Products: Proceedings of the 16th ESACT Meeting April 25-29, 1999, Lugano, Switzer*land*). Then, it is possible to use regular suspension bioreactors (systems derived from bacterial culture, e.g. US10640741 B2). Doing so allows for the volumetric growth of a very large number of cells, not being limited by a surface growth. At the end of the culture, it will be necessary to clear the desired biologic from the culture mix.

However the process for obtaining suspension-adapted cell lines is long and costly, especially since a cell line must be issued from a single original clone, which must be selected, tested and stabilized from the original adherent cell lineage (Adaptation of Recombinant HEK-293 Cells to Growth in Serum Free Suspension. Mcallister R., et al., 1999, Animal Cell Technology: Products from Cells, Cells as Products. Springer, Dordrecht ; Evolution from adherent to suspension: systems biology of HEK293 cell line development, Malm, M., et al.. Sci Rep 10, 18996, 2020). This process may take multiple years to be completed. Once the stable cell line is designed, there remains issues with the suspension culture of adherent cells, namely because these ones tend to aggregate with each other quite easily, which is something to avoid (Scalable Production of AAV Vectors in Orbitally Shaken HEK293 Cells, Blessing D, et al.. Mol Ther Methods Clin Dev. 2018 Nov 22).

Another solution is to use a carrier allowing volumetric culture, in order to reduce the surface footprint for the culture of very large quantities of adherent cells. Providing a growth support in a volumetric approach is something that can be done by attaching cells to micro carriers. For instance US20140356949A1 discloses a carrier for expansion of induced pluripotent stem cells comprising a substrate with one or more outer surfaces modified with gas plasma treatment and one or more structured indentations on the outer surfaces. Such carrier will then be used in a regular suspension bioreactor.

Another approach could be to attach the cells to a 2.5 D growth support such as a fibrous matrix or a polymer foam, a fractal scaffold that offers a lot of surface in a 3D space (Valkama, A. et al., Optimization of lentiviral vector production for scale-up in fixed-bed bioreactor, Nature, Gene Therapy 25, 39-46, 2018).

However with the use of micro carriers, or with a solid substrate, a volumetric loss is induced by the presence of said substrate. For an equal volume, a carrier based culture will yield lower titers than its suspension equivalent because of this, lessening the improvement. Moreover, the use of a support for adherent cell lines often induces inhomogeneities in gases and nutrients distribution in the case of perfusion bioreactors, and a loss in culture density due to the space taken by the growth support.

The existing solutions described above are oriented towards the use of the already developed bioreactors inherited from the culture of monocellular microorganisms. However, mammalian cells are fundamentally different, and require a very controlled environment. Especially, the shear rates that exist in the use of regular stirred tanks bioreactors is crucial for the good distribution and mixing of gases and nutrients but is very detrimental to these cell lines.

In the past years, reactors using acoustic devices have been developed as they allow holding particles or cells in a reactor. For instance patent application US2016/0369236 A1 particularly discloses a process for continuously collecting cells from a cell culture, the process being performed in a bioreactor including a device for producing multi-dimensional standing waves, which are used to hold a cell culture in place. A nutrient fluid stream is circulated through the bioreactor past the cell culture to collect biological products produced be the cell culture. Patent applications US20180298323 A1 and WO2019140019 A1 disclose acoustic devices comprising an acoustic chamber or reactor and an ultrasonic transducer to create a multi-dimensional acoustic standing wave in the acoustic chamber which help holding particles or cells. Patent application US20170175073 A1 discloses a system comprising a bioreactor, a primary clarification stage with an acoustophoretic separator downstream of and fluidly connected to the bioreactor. In such system, the acoustophoretic separator comprises a flow chamber, an ultrasonic transducer an opposed reflector. These allow producing a multi-dimensional standing wave in the flow chamber.

In the above state of the art, acoustic actuation is generally in direct connection with the volume in which the biologics of interest are cultivated. To be able to culture cells at a high density and for a long period of time, then a renewal of culture medium and gases must take place. However this imposes a condition on the strength of the acoustic field, as any fluid circulation will have to be counteracted by the acoustic field for its benefits to stay. Moreover, acoustic actuation of a liquid layer generates in said liquid a large-scale recirculation commonly named acoustic streaming (Jacob S. Bach and Henrik Bruus, Bulk-driven acoustic streaming at resonance in closed microcavities, Phys. Rev. E 100, 023104, 7 August 2019). This fluid motion can induce additional shear on the fragile cells, and worse, cause them to fall out of their acoustic position if the acoustic field is not strong enough.

There is therefore the need for a system and a method thereof which facilitate the renewal of the culture medium and gases without generating additional shear or any other mechanism which would affect the cultured cells. The invention seeks to overcome the aforementioned drawbacks of the prior art as it aims to prevent any disruption in the environment in which the cells are cultured while providing a scalable technology for the culture of cells and the production of biologics.

SUMMARY OF THE INVENTION

To this effect the invention discloses a cell culture system comprising: a culture chamber comprising: a flow compartment and a culture compartment being separated by a membrane, wherein the membrane is permeable to gas and is acoustically transparent; at least one inlet and one outlet to the flow compartment; at least one inlet and one outlet to the culture compartment; an acoustic actuation device comprising at least a first element being an ultrasonic transducer and an opposed second element being a reflector or a second ultrasonic transducer, wherein the acoustic actuation device is arranged to generate an acoustic field in the culture compartment in order to maintain cells in the culture compartment.

Advantageously, the culture compartment of the cell culture system comprises a first end, a second end opposed to the first end, and a main section between the first end and the second end, wherein the main section of the culture compartment is at least partially situated inside the inner volume of the flow compartment.

Advantageously, the culture compartment of the cell culture system is entirely situated inside the inner volume of the flow compartment.

Advantageously, the distance between the acoustic actuation device and the inner volume of the flow compartment ranges from 500 μm to 5 mm.

Advantageously, the distance between the first element and the second element of the acoustic actuation device follows the equation (1) being:

$$h_{tot} = n\frac{\lambda}{2} = n\frac{c}{2f} \qquad (1)$$

wherein $h_{tot}$ is the distance between the first and the second element of the acoustic actuation device, $f$ is the acoustic frequency used, cis the propagation speed of the acoustic wave in the liquids between the first and second element of the acoustic actuation device, $\lambda$ is the wavelength of the acoustic wave.

Advantageously, the membrane of the cell culture system is permeable to nutrients.

Advantageously, the walls of the culture chamber of the cell culture system are at least partially constituted by the acoustic actuation device.

Advantageously, the membrane of the cell culture system comprises polyethylene glycol and/or polydimethylsiloxane.

Advantageously, the cell culture system comprises a sampling inlet.

Advantageously, the acoustic actuation device of the cell culture system further comprising a first element comprising a plurality of ultrasonic transducers and an opposed second element comprising a plurality of ultrasonic transducers and/or reflectors, and the plurality of ultrasonic transducers of the first element and the plurality of ultrasonic transducers and/or reflectors of the second element are arranged to generate an acoustic field and to maintain cultured cells in the culture compartment at at least two locations which are separated from each other by a distance ranging from 50 to 500 $\mu$m. The separated cultured cells in the culture compartment may correspond to separated aggregates of cultured cells.

The invention also discloses a set of cell culture systems as defined in any of the previous claims, the systems being parallelized.

The invention also discloses a method for culturing cells using the cell culture system as described previously, the method comprising: flowing a first liquid from the inlet of the flow compartment to the outlet of the flow compartment of the culture chamber and flowing a second liquid from the inlet of the culture compartment to the outlet of the culture compartment of the culture chamber; generating an acoustic field in the culture compartment with the acoustic actuation device; introducing at least one cell in the culture compartment of the culture chamber; and maintaining the at least one cell introduced in the culture compartment.

Advantageously, the excitation frequencies of the ultrasonic transducer of the acoustic actuation device in the method for culturing cells is in the range from 500 kHz to 15 MHz and the excitation amplitude is in the range from 1 to 30 W.

Advantageously, the method for culturing cells further comprises temporarily setting the acoustic actuation device so as to induce cavitation at the location where the at least one cell is maintained.

Advantageously, temporarily setting the acoustic actuation device so as to induce cavitation comprises decreasing the frequency of the acoustic actuation device to a range of 2 to 3 MHz and increasing the amplitude to a range from 200 to 400 kHz.

BRIEF DESCRIPTION OF THE DRAWINGS

The invention will be better understood and its various characteristics and advantages will emerge from the following description of a number of exemplary embodiments and its appended figures in which:

- Figure 1 displays a cross-section of a cell culture system comprising an acoustic actuation device and a cell chamber comprising a flow compartment and a culture compartment, and;
- Figure 2 displays a cross-section of a cell culture system comprising an acoustic actuation device and a cell chamber comprising a flow compartment and a culture compartment, wherein the main section of the culture compartment is situated inside the inner volume of the flow compartment;
- Figure 3 displays a cell culture system comprising an acoustic actuation device and a cell chamber comprising a flow compartment and a culture compartment, wherein the culture compartment is entirely situated inside the inner volume of the flow compartment;
- Figure 4 displays a cell culture system comprising an acoustic actuation device and a cell chamber comprising a flow compartment and multiple culture compartments.

DETAILED DESCRIPTION OF THE INVENTION

In this specification, the invention will be described by way of examples. However, the invention is not restricted to these examples.

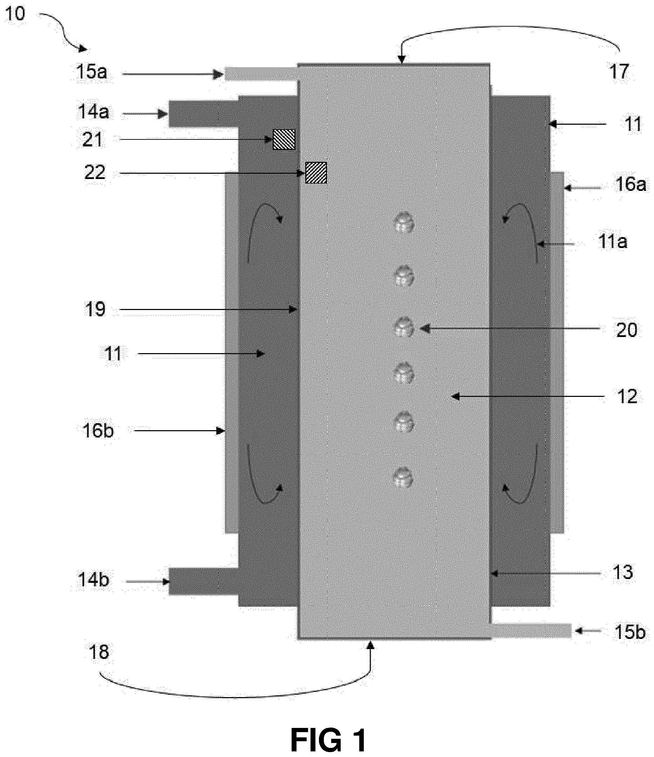

Figure 1 displays a cell culture system 10 comprising a culture chamber comprising a flow compartment 11 and a culture compartment 12, the culture compartment having a first end 17, an opposed second end 18 and a main section 19 between the first end and the second end. A membrane 13 which is gas permeable separates the flow compartment 11 from the culture compartment 12. The culture chamber of figure 1 also comprises an inlet 14a and an outlet 14b to the flow compartment and an inlet 15a and an outlet 15b to the culture compartment. The cell culture system also comprises an acoustic actuation device which comprises a first element 16a which is an ultrasonic transducer and an opposed second element 16b which is a reflector. The first and the second elements of the acoustic actuation device are arranged so as to be able to generate an acoustic field in the culture compartment. Such an acoustic field allows maintaining cells 20 in the culture compartment 12, the cells will adhere progressively to each other and thus provide each other with the necessary growth support. In figure 1, the first and second elements of the acoustic actuation device are fixed on the flow compartment although they may be located elsewhere, for instance on the first end and the second end of the culture compartment. Preferably, the elements of the acoustic actuation device are arranged so as to generate an acoustic field in the center of the culture compartment, e.g. in the center of gravity, so as for the cells 20 to be maintained in the culture compartment 12 without adhering to the membrane 13 and therefore being maintained in suspension.

The cells in the culture compartment, not being bound to a mechanical point, may be manipulated in the volume of the culture compartment by adjusting the phase of the acoustic wave generated by the acoustic actuation device. For instance, adjusting the phase of the acoustic wave allows moving the cells to a sampling outlet, which may correspond to the inlet 15a or the outlet 15b or to another outlet connected to the volume of the culture compartment (not shown).

By flow compartment and culture compartment in the present invention, it is meant a volume physically delimited by walls or, at the separation between the flow compartment and the culture compartment, delimited by a membrane. Although it is preferred that the separation between the flow compartment and the culture compartment is constituted by the membrane 13, such separation may partially comprise walls. Preferably, and this is not limited to the cell culture system of figure 1, these walls are biocompatible, do not release particles in the liquid with which they are in contact, are acoustically transparent, and are manufacturable by injection or moulding. For instance, such walls may be made of materials comprising polypropylene or polystyrene, or being obtained from polymer resin adapted for stereolithography.

The inlet 14a allows introducing a liquid in the flow compartment 11 which will then exit through the outlet 14b. The liquid to introduce in the flow compartment may comprise gases or nutrients and combinations thereof. The inlet 15a allows introducing a liquid in the culture compartment 12 which will then exit through the outlet 15b. The liquid to introduce in the culture compartment may comprise gases or nutrients and combinations thereof. In particular, the inlet 15a of the culture compartment 12 allows introducing one or more cells in the culture compartment. The outlet 15b of the culture compartment 12 allows collecting the one or more cells which were introduced previously and which may have been maintained and cultured in the culture compartment. By "cultured cell", it is meant a cell which has been maintained in the culture compartment 12 and which may have undergone for instance multiplication, differentiation or maturation.

In figure 1, the culture compartment 12 has a first end 17, a second end 18 opposed to the first end and a main section 19 between the first end and the second end. The main section 19 of the culture compartment 12 is partially situated inside the inner volume 11b of the flow compartment 11 while the first end 17 and the second end 18 of the culture compartment are not situated inside the inner volume of the flow compartment. Preferably, the exchange surface between the culture compartment and the flow compartment, which corresponds to the membrane separating these compartments, is the largest possible so as to maximize the exchange between the liquids in the flow compartment and in the culture compartment.

In figure 1, a first group of sensors 21 is situated in the flow compartment 11 and a second group of sensors 22 is situated in the culture compartment 12. These groups of sensors may be placed in different locations in the corresponding compartment. Alternatively, the first and/or second groups of sensors may be situated in the inlet and/or outlet of the flow or culture compartments. Further, the groups of sensors may be connected to one or more controllers which are situated outside the compartments. The connection between the groups of sensors and the controllers may comprise wires or may be wireless. The groups of sensors 21, 22 may be able to monitor pH, temperature, dissolved oxygen, carbon dioxide, pressure, flux rate, shearing rate, etc.

The cell culture system 10 of figure 1 allows introducing a liquid comprising at least one cell 20 in the culture compartment 12 via the inlet 15a and maintaining the at least one cell inside the culture compartment with the first and second elements 16a, 16b of the acoustic actuation device. The at least one cell 20 is provided with gases for its culture, differentiation or maturation with the liquid which is fed through inlet 14a of the flow compartment, the gases passing through the membrane 13. The membrane 13 prevents the turbulences 11a, generated in the flow compartment 11 by the feeding of a liquid through inlet 14a, from being transmitted in the culture compartment 12. Indeed, the turbulences 11a would generate shearing in the environment of the cultured cells, causing mechanical or biological stress to the cells and impacting their multiplication, differentiation or maturation. In the worst case, and depending on the cells to be cultured, shear would cause several cells to die. Notably, the cell culture system of the present invention does not need impellers or blades for mixing the content of the culture compartment.

Therefore a liquid introduced through inlet 14a at a feeding rate which would generate a turbulent flow may still be introduced in the flow compartment, the membrane preventing the turbulences from being transmitted to the culture compartment. Moreover, as the feeding rate is not limited to the turbulences which may be generated, the renewal of the liquid in the flow compartment may be carried out faster and the gas supplied to the culture compartment may be greater and regulated more finely and/or faster. In addition, the liquid introduced into the flow compartment may comprise bubbles without causing harm to the cells. Bubbles may be introduced in the flow compartment for instance within a liquid introduced through the inlet of the flow compartment. Bubbles may increase the gas exchanges between a liquid introduced in the flow compartment and the liquid already present in the flow compartment. Accordingly, the increase of gas exchanges within the flow compartment will also increase gas exchanges between the flow compartment and the culture compartment. Usually, bubbles when conducting acoustic manipulation are a must-avoid because they generate high shear when oscillating under the acoustic actuation. However, according to the cell culture system of the present invention, the membrane prevents any shear from being transmitted to the culture compartment and to the cells.

Furthermore, as the renewal of the liquid in the flow compartment may be carried faster, the control of the temperature, the pH, the dissolved oxygen or carbon dioxide and concentrations of chemicals in the flow compartment and in the culture compartment may be regulated more finely and/or faster. The culture system of the present invention allows renewing the gas content in the liquid in the culture compartment while avoiding changing the flux rate in the culture compartment.

Although not represented in figure 1, in three dimensions the cell culture system of figure 1 and particularly the culture chamber may have a global shape of a right circular cylinder, a square cylinder or any other shapes. The culture chamber may also have a cuboid shape such as a rectangular cuboid, so as to comprise flat and parallel surfaces on which the acoustic actuation device may be fixed.

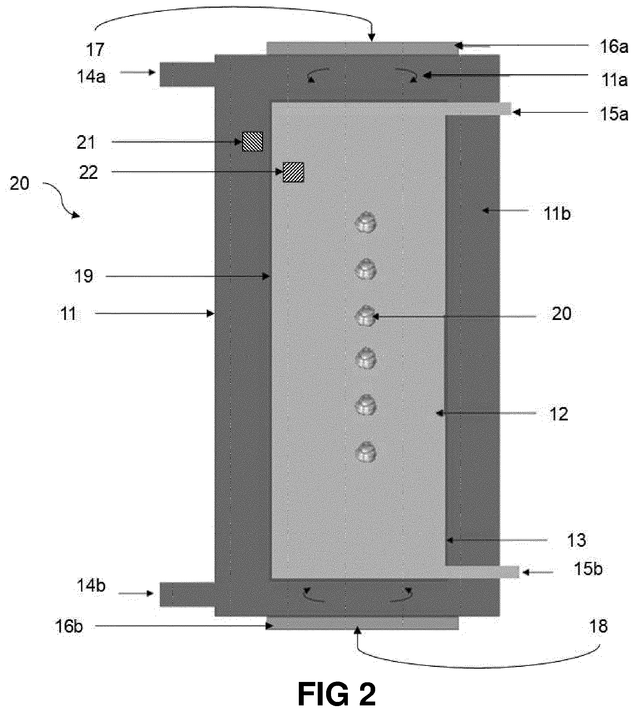

Figure 2 displays a cell culture system 20 comprising a culture chamber comprising a flow compartment 11 and a culture compartment 12, the culture compartment having a first end 17 and an opposed second end 18 and a main section 19 between the first end and the second end of the culture compartment. A membrane 13 which is gas permeable separates the flow compartment 11 from the culture compartment 12. The culture chamber of figure 2 also comprises an inlet 14a and an outlet 14b to the flow compartment and an inlet 15a and an outlet 15b to the culture compartment. The cell culture system also comprises an acoustic actuation device which comprises a first element 16a which is an ultrasonic transducer and an opposed second element 16b which is a reflector. The first and the second elements are arranged so as to be able to generate an acoustic field in the culture compartment. In figure 2, the first end, the second end and the main section of the culture compartment are situated inside the inner volume 11b of the flow compartment 11. The cell culture system also comprises similar groups of sensors 21 and 22 as in figure 1, the groups being situated in the flow compartment 11 and in the culture compartment 12.

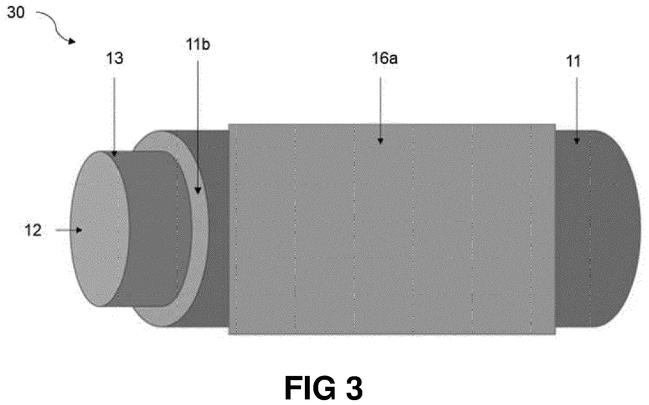

Figure 3 displays a cell culture system 30 comprising an acoustic actuation device comprising a first element 16a and an opposed second element (not visible) and a cell chamber comprising a flow compartment 11 and a culture compartment 12, wherein the culture compartment is entirely situated inside the inner volume 11b of the flow compartment 11. Figure 3 only displays a part of the cell culture system so as to illustrate the culture compartment inside the flow compartment. A membrane 13 separates the volume of the flow compartment and the volume of the culture compartment. In figure 3, the flow compartment has the shape of a right circular cylinder which surrounds the culture compartment which also has the shape of a right circular cylinder, the flow compartment and the culture compartment sharing the same axis. Accordingly, the flow compartment 11 may be provided with a liquid comprising gases so as for the gases to be transmitted via the membrane 13 to the liquid in the culture compartment 12 so as to supply the cells.

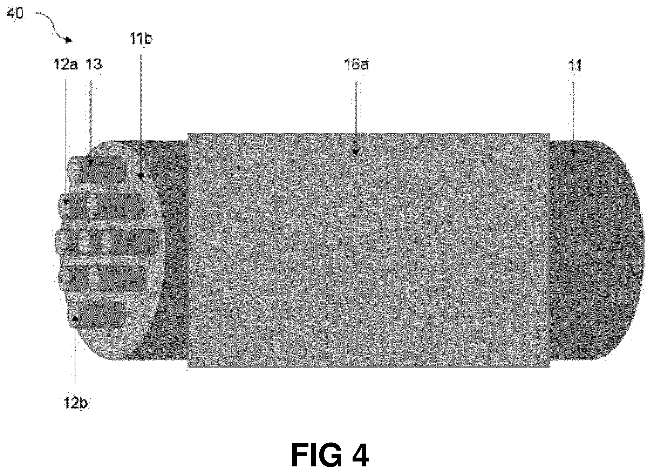

Figure 4 displays a cell culture system 40 comprising an acoustic actuation device comprising a first element 16a and an opposed second element (not visible) and a cell chamber comprising a flow compartment 11 and a culture compartment 12, wherein the culture compartment comprises multiple right circular cylinders having their axis parallel to the axis of the flow compartment. All the right circular cylinders of the culture compartment are entirely situated inside the inner volume 11b of the flow compartment 11. A membrane 13 separates the volume of the flow compartment and the volume of the multiple right circular cylinders of the culture compartment. In figure 4, the flow compartment surrounds the multiple right circular cylinders of the culture compartment. Such embodiment allows growing different type of cells at the same time, while supplying the gas necessary for the cell culture with only the liquid provided in the flow compartment. Each right circular cylinder of the culture compartment may be connected to an inlet and an inlet or they may share the same inlet and outlet.

The membrane 13 in figures 1 to 4 is acoustically transparent, meaning that its acoustic impedance is the same as the liquid in the culture compartment. In particular embodiments, the membrane is also permeable to nutrients, which allows introducing nutrients in the flow compartment so as they diffuse in the culture compartment through the membrane. The membrane may be flexible and therefore the culture compartment may be a bag suspended in the inner volume of the flow compartment. Preferably, the membrane 13 is made of biocompatible materials. Membrane 13 may comprise polyethylene glycol and/or polydimethylsiloxane (PDMS) or may consist in polyethylene glycol and/or polydimethylsiloxane (PDMS).

Preferably, the distance between the first element and the second of the acoustic actuation device is dependent on the acoustic frequency used and follows the equation (1):

$$h_{tot} = n\frac{\lambda}{2} = n\frac{c}{2f} \qquad (1)$$

wherein $h_{tot}$ is the distance between the first and the second element of the acoustic actuation device, $f$ is the acoustic frequency used, $c$ is the propagation speed of the acoustic wave in the liquids between the first and second element of the acoustic actuation device, $\lambda$ is the wavelength of the acoustic wave. When the distance between the first element and the second of the acoustic actuation device follows equation (1) the acoustic waves generated by the ultrasonic transducers and the waves reflected by the reflectors may resonate with an improved precision so as to generate an acoustic field to maintain the cells in the culture compartment with a finer control of the acoustic field. Also, when the distance between the first element and the second of the acoustic actuation device follows equation (1), the acoustic waves generated by the ultrasonic transducers and the waves reflected by the reflectors may have increased amplitude, reducing the need for actuation of the piezoelectric transducers.

Preferably, the thickness of the walls of the flow compartment which are contiguous with the ultrasonic transducer of the acoustic actuation device is dependent on the acoustic frequency used and follows the equation (2):

$$h_{wall} = (2n + 1)\frac{\lambda}{4} = (2n + 1)\frac{c}{4f} \qquad (2)$$

wherein $h_{wall}$ is the thickness of the walls of the flow compartment which are contiguous with the ultrasonic transducer of the acoustic actuation device, $f$ is the acoustic frequency used, $c$ is the propagation speed of the acoustic wave in the walls of the flow compartment, $\lambda$ is the wavelength of the acoustic wave. When the thickness of the walls of the flow compartment follows equation (2), the acoustic waves generated are transmitted through the walls without being impacted, which allows a finer control of the acoustic field generated to maintain the cells in the culture compartment.

Preferably, the cell culture system of the invention comprises a total volume of a flow compartment and a culture compartment of 1 milliliter to 1 000 liters, even more preferably from 200 liters to 1 000 liters. A culture system of a total volume of 1 mL to 250 mL is particularly adapted for optimizing the process, for parametric exploration or paralleling experiments. A culture system of a total volume of 1 L to 80 L is particularly adapted for preclinical production, animal testing or small therapies. A culture system of a total volume over 150 L is particularly adapted for industrial production and clinical trials gene therapy.

Preferably, the ultrasonic transducers of the present invention comprise piezoelectric ceramics, such as PZT ceramics although the invention is not limited to PZT ceramics. According to the system of the invention, excitation frequencies in the range from 100 kHz to 20 MHz and preferably from 500 kHz to 15 MHz are applied by the ultrasonic transducer. Further, the excitation amplitude is in the range from 1 to 1000 W. In particular, the excitation amplitude is at minimum of 1,5 W per liter. Therefore in the case of a cell culture system of 1 liter, the excitation amplitude is from 1,5 to 30W, in the case of a cell culture of 10 L, the excitation amplitude is from 15 to 200W and in the case of a cell culture of 100 L, the excitation amplitude is from 150 to 1000 W. The ultrasonic transducers of the acoustic actuation device may also be flexible so that they may adapt to the shape of the flow compartment of the cell culture system.

For instance, the typical size of an ultrasonic transducer of the first or second element of the acoustic actuation device is of 70 x 70 millimeter, the thickness depending on the frequency, being of 0.5 mm for a frequency of 4 MHz, 1 mm for a frequency of 2 MHz and 2 mm for a frequency of 1 MHz.

The reflectors of the second element of the acoustic actuation device have a significant impedance break with the culture medium in the culture compartment. Materials with a high acoustic impedance are therefore preferred, such as steel with a density of 8 $g/cm^3$, speed of sound of 5521 m/s and an acoustic impedance of 44,2 MPa·s/$m^3$, for instance stainless steel 316 or steel which are used in the biomedical industry, or such as aluminum with a density of 2,7 $g/cm^3$, speed of sound of 6108 m/s and an acoustic impedance of 16,5 MPa·s/$m^3$.

Preferably, the thickness of the membrane ranges from 50 $\mu$m to 2 mm. Preferably, the size of the pores of the membrane ranges from 0,2 to 4 $\mu$m, which prevents the cultured cells from exiting the culture compartment.

Preferably, the sensors are configured to monitor multiple parameters and to warn if a threshold of a parameter has been exceeded. For instance, the pH should range from 6 to 8, the temperature from 30 to 40°C, the dissolved oxygen from 0 to 20 mg/L, the carbon dioxide from 0 to 100 mg/L, the pressure from 0 to 1 bar and the flux rate from 0 to 20 L/min.

In a particular embodiment, which may be combined with the other embodiments disclosed herein, the first element and/or the second element of the acoustic actuation device may be integrated in the walls of the culture chamber, e.g. in the walls of the flow compartment or of the culture compartment. Alternatively, the acoustic actuation device may constitute a part of the walls of the flow compartment or of the culture compartment.

The cells to be cultured in the cell culture system of the invention may be bacteria, e.g. cyanobacteria, plant cells, e.g. algae, animal cells, e.g. insect cells or mammalian cells (human or non-human) such as mesenchymal stem cells, embryonic stem cells, induced pluripotent stem cells, hematopoietic stem cells, T-lymphocytes (cart-T), Human Embryonic Kidney cells (HEK) or others. In the case of human embryonic stem cells, these are obtained only without destruction of the embryo from which they are derived and are not suitable for inducing the developmental process of a human being.

The system of the invention allows enabling the cells to grow in volume, to save time for their multiplication and to optimize it, while avoiding the need for growth support consumables.

The system of the invention may be associated to other similar systems of the invention as a set of systems, the systems being parallelized in order to increase the global capacity of the overall set of systems. In a preferred embodiment these systems have a rectangular cuboid shape so that they may be stacked, allowing them, inter alia, to occupy as minimal space as possible. In a set of systems, the groups of sensors may be connected to one or more controller to supervise the overall set of systems or a particular system among the systems of the set of systems.

Example

In a particular example, the cell culture system comprises an acoustic actuation device comprising a first element comprising 3 ultrasonic transducers and an opposed second element comprising 3 reflectors, each reflector being opposed to an ultrasonic transducer so as to be able to generate an acoustic field in the culture compartment. The ultrasonic transducers and the reflectors size is 70 x 70 mm and are arranged one after the other to cover a length of 210 mm. The thickness of the walls of the flow compartment follows the equation (3):

$$h = (2n + 1)\frac{\lambda}{4} = (2n + 1)\frac{c}{4f} \qquad (3)$$

wherein $h$ is the thickness of the thickness of the walls of the flow compartment, $f$ is the acoustic frequency used, $c$ is the propagation speed of the acoustic wave in the walls of the flow compartment, $\lambda$ is the wavelength of the acoustic wave in the material composing the walls.

In this example, the membrane is made of polydimethylsiloxane (PDMS) which prevents the transmission of liquid turbulence from the flow compartment to the culture compartment, but other materials may be used for the membrane.

**Claims**

1. A cell culture system (10) comprising:

   a culture chamber comprising:

      - a flow compartment (11) and a culture compartment (12) being separated by a membrane (13), wherein the membrane is permeable to gas and is acoustically transparent;
      - at least one inlet (14a) and one outlet (14b) to the flow compartment;
      - at least one inlet (15a) and one outlet (15b) to the culture compartment;

      an acoustic actuation device comprising at least a first element (16a) being an ultrasonic transducer and an opposed second element (16b) being a reflector or a second ultrasonic transducer,
      wherein the acoustic actuation device is arranged to generate an acoustic field in the culture compartment in order to maintain cells in the culture compartment.

2. The cell culture system (10) of claim 1, the culture compartment (12) comprising a first end (17), a second end (18) opposed to the first end, and a main section (19) between the first end and the second end, wherein the main section of the culture compartment is at least partially situated inside the inner volume (11b) of the flow compartment (11).

3. The cell culture system (20) of claim 2, wherein the culture compartment (12) is entirely situated inside the inner volume (11b) of the flow compartment (11b).

4. The cell culture system (10) of any of the previous claims, wherein the distance between the acoustic actuation device and the inner volume of the flow compartment ranges from 500 $\mu$m to 5 mm.

5. The cell culture system (10) of any of the previous claims, wherein the distance between the first element (16a) and the second element (16b) of the acoustic actuation device follows the equation (1) being:

$$h_{tot} = n\frac{\lambda}{2} = n\frac{c}{2f} \qquad (1)$$

wherein $h_{tot}$ is the distance between the first and the second element of the acoustic actuation device, $f$ is the acoustic frequency used, $c$ is the propagation speed of the acoustic wave in the liquids between the first and second element of the acoustic actuation device, $\lambda$ is the wavelength of the acoustic wave.

6. The cell culture system (10) of any of the previous claims, wherein the membrane (13) is permeable to nutrients.

7. The cell culture system (10) of any of the previous claims, wherein the walls of the culture chamber are at least partially constituted by the acoustic actuation device.

8. The cell culture system (10) of any of the previous claims, wherein the membrane (13) comprises polyethylene glycol and/or polydimethylsiloxane.

9. The cell culture system (10) of any of the previous claims, wherein the system comprises a sampling inlet.

10. The cell culture system (10) of any of the previous claims, the acoustic actuation device further comprising a first element comprising a plurality of ultrasonic transducers (16a, 16c, 16e, 16g) and an opposed second element comprising a plurality of ultrasonic transducers and/or reflectors (16b, 16d, 16f, 16h), and wherein the plurality of ultrasonic transducers of the first element and the plurality of ultrasonic transducers and/or reflectors of the second element are arranged to generate an acoustic field and to maintain cultured cells (20) in the culture compartment (12) at at least two locations which are separated from each other by a distance ranging from 50 to 500 $\mu$m.

11. A set of cell culture systems as defined in any of the previous claims, the systems being parallelized.

12. A method for culturing cells using the cell culture system (10) of any of the previous claims, the method comprising:

- flowing a first liquid from the inlet (14a) of the flow compartment (11) to the outlet (14b) of the flow compartment of the culture chamber and flowing a second liquid from the inlet (15a) of the culture compartment (12) to the outlet (15b) of the culture compartment of the culture chamber;
- generating an acoustic field in the culture compartment with the acoustic actuation device;
- introducing at least one cell in the culture compartment of the culture chamber; and
- maintaining the at least one cell introduced in the culture compartment.

13. The method of claim 12, wherein the excitation frequencies of the ultrasonic transducer of the acoustic actuation device is in the range from 500 kHz to 15 MHz and the excitation amplitude is in the range from 1 to 30 W.

14. The method of one of the previous claims, wherein the method further comprises temporarily setting the acoustic actuation device so as to induce cavitation at the location where the at least one cell is maintained.

15. The method of claim 14, wherein temporarily setting the acoustic actuation device so as to induce cavitation comprises decreasing the frequency of the acoustic actuation device to a range of 2 to 3 MHz and increasing the amplitude to a range from 200 to 400 kHz.

**FIG 1**

17
14a
21
20
22
19
11
14b
16b

16a
11a
15a
11b
20
12
13
15b
18

## FIG 2

**FIG 3**

**FIG 4**

Europäisches
Patentamt

European
Patent Office

Office européen
des brevets

# EUROPEAN SEARCH REPORT

Application Number

EP 21 30 6217

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A,D | WO 2019/140019 A1 (FLODESIGN SONICS INC [US]) 18 July 2019 (2019-07-18)<br>* page 14 - page 15 *<br>* page 20 - page 21 *<br>* figures 3A,3B,6A-6C * | 1-15 | INV.<br>C12M1/00<br>C12M1/26<br>C12M1/42 |
| A | EP 3 360 955 A1 (FLODESIGN SONICS INC [US]) 15 August 2018 (2018-08-15)<br>* paragraph [0066] - paragraph [0069]; figures 5,6 * | 1-15 | |
| A | EP 1 121 555 A1 (FEDERAL MOGUL TECHNOLOGY LTD [GB]) 8 August 2001 (2001-08-08)<br>* page 7, line 2 - page 10, line 10; figures 1-3 * | 1-15 | |
| A | US 2019/292510 A1 (TANDON VISHAL [US] ET AL) 26 September 2019 (2019-09-26)<br>* paragraph [0100] - paragraph [0106]; figures 5D,5E * | 1-15 | |

TECHNICAL FIELDS
SEARCHED (IPC)

C12M

The present search report has been drawn up for all claims

| Place of search | Date of completion of the search | Examiner |
|---|---|---|
| Berlin | 18 March 2022 | Cubas Alcaraz, Jose |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

 

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 21 30 6217

This annex lists the patent family members relating to the patent documents cited in the above-mentioned European search report.
The members are as contained in the European Patent Office EDP file on
The European Patent Office is in no way liable for these particulars which are merely given for the purpose of information.

18-03-2022

| Patent document cited in search report | | Publication date | Patent family member(s) | | Publication date |
|---|---|---|---|---|---|
| WO 2019140019 | A1 | 18-07-2019 | CN | 111630146 A | 04-09-2020 |
| | | | EP | 3737738 A1 | 18-11-2020 |
| | | | WO | 2019140019 A1 | 18-07-2019 |
| EP 3360955 | A1 | 15-08-2018 | CA | 2994643 A1 | 14-08-2018 |
| | | | CN | 108424851 A | 21-08-2018 |
| | | | EP | 3360955 A1 | 15-08-2018 |
| EP 1121555 | A1 | 08-08-2001 | AT | 234442 T | 15-03-2003 |
| | | | AU | 6215599 A | 01-05-2000 |
| | | | DE | 69905920 T2 | 30-10-2003 |
| | | | EP | 1121555 A1 | 08-08-2001 |
| | | | ES | 2189497 T3 | 01-07-2003 |
| | | | GB | 2342708 A | 19-04-2000 |
| | | | PT | 1121555 E | 30-06-2003 |
| | | | US | 6491067 B1 | 10-12-2002 |
| | | | WO | 0022340 A1 | 20-04-2000 |
| US 2019292510 | A1 | 26-09-2019 | US | 2019292510 A1 | 26-09-2019 |
| | | | WO | 2019183239 A1 | 26-09-2019 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 10640741 B2
- US 20140356949 A1
- US 20160369236 A1
- US 20180298323 A1
- WO 2019140019 A1
- US 20170175073 A1

### Non-patent literature cited in the description

- **BERG DT et al.** High-level expression of secreted proteins from cells adapted to serum-free suspension culture. *Biotechniques,* June 1993
- **MCALLISTER, R et al.** Adaptation of Recombinant HEK-293 Cells to Growth in Serum Free Suspension, Animal Cell Technology: Products from Cells. *Cells as Products: Proceedings of the 16th ESACT Meeting,* 25 April 1999
- **MCALLISTER R. et al.** Animal Cell Technology: Products from Cells, Cells as Products. Springer, Dordrecht. *Evolution from adherent to suspension: systems biology of HEK293 cell line development,* 1999
- **MALM, M. et al.** *Sci Rep,* 2020, vol. 10, 18996
- **BLESSING D et al.** Scalable Production of AAV Vectors in Orbitally Shaken HEK293 Cells. *Mol Ther Methods Clin Dev,* 22 November 2018
- **VALKAMA, A et al.** Optimization of lentiviral vector production for scale-up in fixed-bed bioreactor. *Nature, Gene Therapy,* 2018, vol. 25, 39-46
- **JACOB S. BACH ; HENRIK BRUUS.** Bulk-driven acoustic streaming at resonance in closed microcavities. *Phys. Rev. E,* 07 August 2019, vol. 100, 023104